# EUROPÄISCHE PATENTANMELDUNG

(11) **EP 0 894 793 A1**
(43) Veröffentlichungstag der Anmeldung: **03.02.1999**
(21) Anmeldenummer: 98810704.1
(22) Anmeldetag: 21.07.1998
(51) Int. Cl.: C07D 277/74, C07D 285/12, C10L 1/24, C10M 135/36

(54) **Phosphorfreie multifunktionelle Schmiermitteladditive**

(30) Priorität: 30.07.1997 CH 1823/97
(71) Anmelder: Ciba Specialty Chemicals Holding Inc., 4057 Basel (CH)
(72) Erfinder: Camenzind, Hugo, 3014 Bern (CH); Dratva, Alfred, 4103 Bottmingen (CH); Hänggi, Peter, 1735 Giffers (CH)

(57) **Zusammenfassung**

Als Schmiermitteladditive geeignet sind die Verbindungen der Formeln 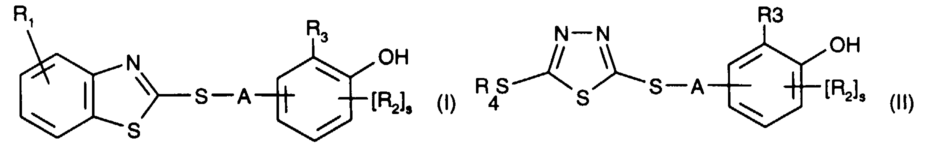 worin die Substituenten und Gruppen folgende bevorzugte Bedeutungen haben:
R₁ = H; R₂ = Methyl oder tert-Butyl; R₃ = tert-Butyl;
s = 0, 1 oder 2;
R₄ = -A-[(C₆H₂)(Methyl oder tert.-Butyl)(OH)(tert.-Butyl)];
A = -CH₂CH₂O-(C=O)-CH₂CH₂- oder -CH₂CH(CH₃)-O-(C=O)-CH₂CH₂-.

## Beschreibung

Die Erfindung betrifft als aschefreie Verschleissschutzmittel und Antioxidantien geeignete Verbindungen der Formeln I und II, diese enthaltende Schmierstoffzusammensetzungen sowie deren Verwendung.

Beim Betrieb von Verbrennungsmotoren hat es sich als günstig erwiesen, metall- und damit aschearme, im Hinblick auf Abgaskatalysator-Verträglichkeit auch phosphorarme, Schmierstoffe zu verwenden. Man sucht daher nach metallfreien und phosphorfreien Additiven oder Additivkombinationen, welche der guten Antioxidations- und Verschleissschutzwirkung der Zinkdialkyldithiophosphate nahekommen. Es sind einige Versuche bekannt, chemische Substrukturen, welche als Öladditive verschiedene Funktionen erwarten lassen, intramolekular zu kombinieren.

In der *U.S.-Patentschrift 3,676,449* sind 1,3,4-Thiadiazol-verbrückte Bis-thiomethylenphenole des Typs 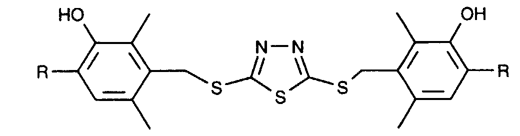 R = tert.- C₄-C₁₂-Alkyl; beschrieben, welche als Antioxidantien für oxydationsempfindliche Materialien, vor allem Polyolefine, geeignet sind.

In der *U.S.-Patentschrift 4,906,393* sind Borate als Reaktionsprodukte von 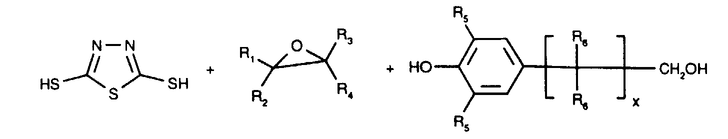 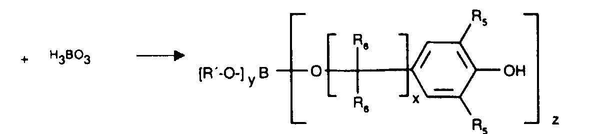 beschrieben, worin R₁, R₂,R₃ und R₄ Wasserstoff oder C₁-C₆₀-Kohlenwasserstoff, gegebenenfalls S, N und/oder O enthaltend; R₅ und R₆ Wasserstoff oder C₁-C₂₀-Kohlenwasserstoff, gegebenenfalls S, N und/oder O enthaltend; R' eine Dimercaptothiadiazol-Einheit; X = 0 - 10 und y + z = 3 bedeuten.

In der *U.S. Patentschrift 5,177,212* sind Umsetzungsprodukte von 2,5-Dimercapto-1,3,4-thiadiazol vom Typ a), b) oder c) mit aliphatischem oder aromatischem Aldehyd und alkyliertem Phenol, im Verhältnis 1:1:1 bis 2:2:1, als Verschleissschutzmittel und Antioxidantien für Schmiermittelzusammensetzungen beschrieben: 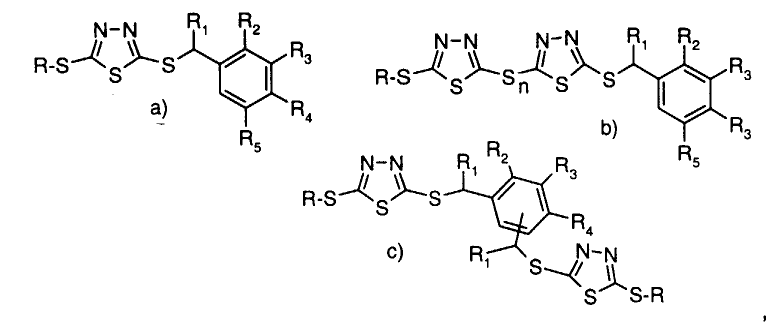 worin R = H, den Terpen-Rest, einen Polymer-Rest (ex Poly-α-olefin oder epoxydiertem Poly-α-olefin); R₁ = H, C₁-C₁₇-Alkyl, Phenyl, Alkyl-subst. Phenyl; R₂ und R₄ = H, OH oder Alkyl, wobei entweder R₂ oder R₄ die Bedeutung von OH hat; R₃ und R₅ = Alkyl bedeuten, und n = 1-2 ist.

Gegenstand der Erfindung sind Verbindungen der im folgenden beschriebenen Formeln I und II, die als asche- und phosphorfreie Verschleissschutz-Additive geeignet sind und zusätzlich eine antioxidative Wirkung haben: 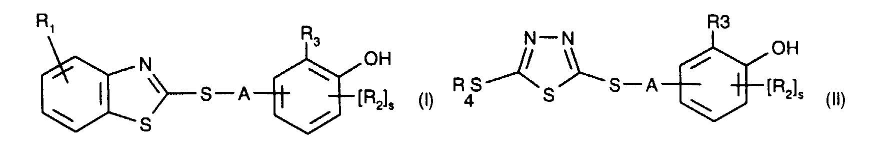 Dabei sind:
R₁ = Wasserstoff oder C₁-C₂₀-Alkyl;
R₂ = Wasserstoff, C₁-C₁₈-Alkyl, C₅-C₁₂-Cycloalkyl, C₇-C₁₀-Bicycloalkyl, Phenyl oder C₇-C₉-Phenylalkyl;
R₃ = C₁-C₁₈-Alkyl, C₅-C₁₂-Cycloalkyl, Phenyl oder C₇-C₉-Phenylalkyl;
s = 0,1 oder 2;
R₄ = C₅-C₂₀-Alkyl, C₅-C₁₂-Cycloalkyl, Phenyl, Naphthyl, C₇-C₉-Phenylalkyl, gegebenenfalls unterbrochen durch ein oder mehrere bivalente Reste aus der Gruppe -O-, -NR₅-, -S-, -C(=O)-O-, -O-C(=O)-, -C(=O)-NR₅- und -NR₅-C(=O)-, oder substituiert durch ein oder mehrere Reste der Gruppe -OH, -NH₂ und -C(=O)-OH oder bedeutet -A-[(C₆H₂)R₂(OH)R₃];
R₅ = H oder C₁-C₁₈-Alkyl;
A = C₁-C₂₅-Alkylen, gegebenenfalls unterbrochen durch ein oder mehrere bivalente Reste aus der Gruppe -O-, -NR₅-, -S-, -C(=O)-O-, -O-C(=O)-, -C(=O)-NR₅- und -NR₅-C(=O)-, oder substituiert durch ein oder mehrere Reste der Gruppe -OH, -NH₂ und -C(=O)-OH, wobei mindestens eine Einheit -C(=O)-O- oder -O-C(=O)- enthalten ist.

Bevorzugt sind:
R₁ = H;
R₂ = H, C₁-C₈-Alkyl, C₅-C₆-Cycloalkyl, Phenyl, Benzyl oder 2-Norbornyl;
R₃ = C₁-C₈-Alkyl, C₅-C₆-Cycloalkyl, Phenyl, Benzyl oder 2-Norbornyl;
s = 0, 1 oder 2;
R₄ = C₅-C₇-Cycloalkyl, Phenyl, Benzyl, C₁-C₁₈-Alkyl, gegebenenfalls unterbrochen durch ein oder mehrere bivalente Reste aus der Gruppe -O-, -NH-, -S-, -C(=O)-O-, -O-C(=O)-,
-C(=O)-NR₅- und -NR₅-C(=O)-, oder substituiert durch ein oder mehrere Reste der Gruppe -OH und -C(=O)-OH oder bedeutet -A-[(C₆H₂)R₂(OH)R₃];
R₅ = H oder C₁-C₈-Alkyl;
A = C₁-C₁₈-Alkylen, gegebenenfalls unterbrochen durch ein oder mehrere bivalente Reste aus der Gruppe -O-, -NR₅-, -S-, -C(=O)-O-, -O-C(=O)-, -C(=O)-NR₅- und -NR₅-C(=O)-, oder substituiert durch ein oder mehrere Reste der Gruppe -OH, -NH₂ und -C(=O)-OH, wobei mindestens eine Einheit -C(=O)-O- oder -O-C(=O)- enthalten ist.

Besonders bevorzugt sind:
R₁ = H; R₂ = Methyl oder tert-Butyl; R₃ = tert-Butyl;
s = 0, 1 oder 2;
R₄ = -A-[(C₆H₂)(Methyl oder tert.-Butyl)(OH)(tert.-Butyl)];
A = -CH₂CH₂O-(C=O)-CH₂CH₂- oder -CH₂CH(CH₃)-O-(C=O)-CH₂CH₂-.

Ganz besonders bevorzugt sind die Verbindungen: 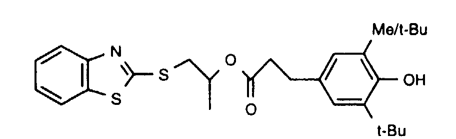 und

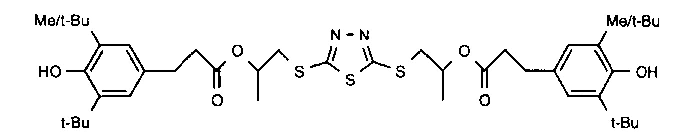

Die Verbindungen sind als multifunktionale Verschleissschutzadditiv mit zusätzlicher antioxydativer Wirkung für Grundöle mit schmierender Viskosität sowie für Kraftstoffe geeignet. Sie sindphosphorfrei und weitgehend aschefrei.

Ein weiterer Gegenstand der Erfindung sind Zusammensetzungen enthaltend eine Verbindung der Formel I oder II oder Mischungen davon in Kombination mit einem Grundöl mit schmierender Viskosität oder Kraftstoffen.

Ein Grundöl mit schmierender Viskosität ist für die Herstellung von schmierenden Fetten oder Schmierstoffen, Metallbearbeitungs-, Getriebe- oder Hydraulikflüssigkeiten verwendbar.

Solche schmierenden Fette oder Schmierstoffe, Metallbearbeitungs-, Getriebe- und Hydraulikflüssigkeiten basieren beispielsweise auf mineralischen oder synthetischen Schmierstoffen oder Ölen oder Mischungen davon. Diese sind dem Fachmann geläufig und in der einschlägigen Fachliteratur, wie beispielsweise in *Chemistry and Technology of Lubricants; Mortier, R.M. and Orszulik, S.T. (Editors); 1992 Blackie and Son Ltd. for GB, VCH-Publishers N.Y. for U.S., ISBN 0-216-92921-0, siehe Seiten 208 ff und 269 ff; in Kirk-Othmer Encyclopedia of Chemical Technology, Fourth Edition 1969, J. Wiley & Sons, New York, Vol. 13, Seite 533 ff. (Hydraulic Fluids); Performance Testing of Hydraulic Fluids; R. Tourret and E.P. Wright, Hyden* & *Son Ltd. GB, on behalf of The Institute of Petroleum London, ISBN 0 85501 317 6; Ullmann's Encyclopedia of Ind. Chem., Fifth Completely Revised Edition, Verlag Chemie, DE-Weinheim, VCH-Publishers for U.S., Vol. A 15, Seite 423 ff (lubricants), Vol. A 13, Seite 165 ff (hydraulic fluids)* beschrieben.

Ferner betrifft die Erfindung ein Verfahren zur Verbesserung der Gebrauchseigenschaften von Schmierstoffen oder Schmierfetten, z.B. Motorenöl, Turbinenöl, Getriebeöl, Hydraulik- oder Metallbearbeitungsflüssigkeiten, oder flüssigen Kraftstoffen (Diesel- oder Ottokraftstoffe), gekennzeichnet durch Zugabe mindestens einer Verbindung der Formel I oder II zum Grundöl oder Kraftstoff zur Erzielung einer verschleissmindernden und/oder antioxydativen Wirkung. Gegenstand der Erfindung ist somit auch die Verwendung von Verbindungen der Formeln I oder II als Additive in Motorenölen, Turbinenölen, Getriebeölen, Hydraulikflüssigkeiten, Metallbearbeitungsflüssigkeiten, Schmierfetten oder Diesel- oder Ottokraftstoffen.

Die Schmierstoffe sind insbesondere Öle und Fette, beispielsweise basierend auf einem Mineralöl. Bevorzugt sind Öle.

Eine weitere Gruppe von Schmierstoffen, die zur Anwendung gelangen können, sind pflanzliche oder tierische Öle, Fette, Talge und Wachse oder deren Gemische untereinander oder Gemische mit den erwähnten mineralischen oder synthetischen Ölen. Pflanzliche und tierische Öle, Fette, Talge und Wachse sind beispielsweise Palmkernöl, Palmöl, Olivenöl, Rüböl, Rapsöl, Leinöl, Erdnussöl, Sojabohnenöl, Baumwollöl, Sonnenblumenöl, Kürbiskernöl, Kokosnussöl, Maisöl, Rizinusöl, Baumnussöl und Mischungen davon, Fischöle, Talge von Schlachttieren wie Rindertalg, Klauenfett und Knochenöl sowie deren modifizierte, epoxidierte und sulfoxidierte Formen, beispielsweise epoxidiertes Sojabohnenöl.

Beispiele von synthetischen Schmierstoffen umfassen Schmierstoffe auf der Basis aliphatische oder aromatische Carboxylester, polymere Ester, Polyalkylenoxide, Poly-α-olefine oder Silicone, Diester zweiwertiger Säuren mit einem einwertigen Alkohol, wie z.B. Dioctylsebacat oder Dinonyladipat, Triester, z.B. von Trimethylolpropan mit einwertigen Säuren oder mit einem Gemisch solcher Säuren, wie z.B. Trimethylolpropantripelargonat, Trimethylolpropan-tricaprylat oder Gemische davon, Tetraester von Pentaerythrit mit einwertigen Säuren oder mit einem Gemisch solcher Säuren, wie z.B. Pentaerythrit-tetracaprylat, oder komplexe Ester von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineralölen z.B. Poly-α-olefine, Schmierstoffe auf Esterbasis, Glycole, Polyglycole und Polyalkylenglycole, sowie deren Mischungen mit Wasser.

Metallbearbeitungsflüssigkeiten und Hydraulikflüssigkeiten können auf Basis der gleichen Substanzen hergestellt werden wie vorstehend für die Schmiermittel beschrieben. Häufig handelt es sich dabei auch um Emulsionen solcher Substanzen in Wasser oder anderen Flüssigkeiten.

Erfindungsgemässe Schmierstoffzusammensetzungen finden z.B. Verwendung in Verbrennungsmotoren, z.B. in Kraftfahrzeugen, ausgerüstet z.B. mit Motoren des Otto-, Diesel-, Zweitakt-, Wankel- oder Orbitaltyps.

Die Verbindungen der Formel I oder II sind gut in Schmierstoffen, Metallbearbeitungs- und Hydraulikflüssigkeiten löslich und sind deshalb als Zusätze zu Schmierstoffen, Metallbearbeitungs- und Hydraulikflüssigkeiten besonders geeignet.

Vorteilhafterweise enthalten die Zusammensetzungen 0,005 bis zu 10,0 Gew.% der Verbindungen der Formel I oder II, bevorzugt 0,01 - 5,0 %, insbesondere 0,01- 0,9 %.

Die Verbindungen der Formel I oder II können den Schmierstoffen auf an sich bekannte Weise beigemischt werden. Die Verbindungen sind beispielsweise in Ölen gut löslich. Es ist auch möglich, mit weiteren Additiven ein Konzentrat oder ein sogenanntes Additivpaket herzustellen, das nach Massgabe des Verbrauchs auf Einsatzkonzentrationen für den entsprechenden Schmierstoff verdünnt werden kann.

Die erfindungsgemässen Zusammensetzungen können zusätzlich weitere Additive enthalten, die zugegeben werden, um ihre Grundeigenschaften noch weiter zu verbessern. Dazu gehören: weitere Antioxidantien, Metallpassivatoren, Rostinhibitoren, Viskositätsindex-Verbesserer, Stockpunkterniedriger, Dispergiermittel, Detergentien, weitere Hochdruck--Zusätze, Antiverschleiss-Additive sowie Reibwertverminderer. Gegebenenfalls können diese Additive synergistisch miteinander oder zusammen mit den erfindungsgemässen Verbindungen wirken. Solche Additive gibt man in den jeweils dafür üblichen Mengen im Bereich von je etwa 0,01 bis 10,0 Gew.% zu. Sollte der Zusatz von phosphor- oder metallhaltigen Additiven weiterhin erforderlich sein, werden diese Additive vorzugsweise in geringen Mengen, z.B. von ca. 0,01 bis 0,1 Gew-%, zugesetzt.

Herstellung: Die Verbindungen der Formeln I und II können über die Umesterung bzw. Veresterung von Carboxylat- bzw. Carbonsäurechlorid-funktionalisierten Alkylphenolen mit Alkohol-funktionalisierten S-Alkyl-mercapto-S/N-Heterozyklen [z.B. Beispiele 2, 3, 5, 7, 8, 10, 12, 13] oder von Carboxylat- bzw. Carbonsäurechlorid-funktionalisierten S-Alkyl-mercapto-S/N-Heterozyklen mit Alkohol-funktionalisierten Alkylphenolen [z.B. Beispiel 14] hergestellt werden.

Es folgen Beispiele für weitere Zusatzstoffe:

### Beispiele für phenolische Antioxidantien:

1.1. Alkylierte Monophenole, z.B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Di-cyclopentyl-4-methylphenol, 2-(1-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Di-nonyl-4-methylphenol, 2,4-Dimethyl-6-(1'-methyl-undec-1'-yl)-phenol, 2,4-Dimethyl-6-(1'-methylheptadec-1'-yl)-phenol, 2,4-Dimethyl-6-(1'-methyl-tridec-1'-yl)-phenol und Mischungen davon.
1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.
1.4. Tocopherole, z.B. α-, β-, γ-, δ-Tocopherol und Mischungen davon (Vitamin E).
1.5. Hydroxylierte Thiodiphenylether, z.B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis-(6-tert-butyl-2-methylphenol), 4,4'-Thio-bis(3,6-di-sec.-amylphenol), 4,4'-Bis(2,6-dimethyl-4-hydroxyphenyl)-disulfid.
1.6. Alkyliden-Bisphenole, z.B. 2,2'-Methylen-bis(6-tert-butyl-4-methylphenol), 2,2'-Methylen-bis(6-tert-butyl-4-ethylphenol), 2,2'-Methylen-bis[4-methyl-6-(a-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(a-methylbenzyl)-4-nonylphenol], 2,2'-Methylen-bis[6-(a,a-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol-bis[3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methyl-benzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)propan, 2,2-Bis(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.
1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterephthalat, Bis(3,5-di-tert-butyl-4-hydroxybenzyl)-sulfid, Isooctyl-3,5-di-tert-butyl-4-hydroxybenzyl-mercaptoacetat.
1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.
1.9. Hydroxybenzyl-Aromaten, z.B. 1,3,5-Tris(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris(3,5-di-tert-butyl-4-hydroxybenzyl)-phenol.
1.10. Triazinverbindungen, z.B. 2,4-Bis-octylmercapto-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.
1.11. Benzylphosphonate, z.B. Dimethyl-2,5-di-tert-butyl-4-hydroxybenzylphosphonat, Di-ethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
1.13. Ester der beta-(3.5-Di-tert-butyl-4-hydroxyphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.14. Ester der beta-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.15. Ester der beta-(3.5-Dicyclohexyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.17. Amide der beta-(3,5-Di-tert-butyl-4-hydroxyphenyl)-propionsäure, wie z.B. N,N'-Bis(3,5-di-tert-butyl-4-hydroxyphenylpropionyl)-hexamethylendiamin, N,N'-Bis(3,5-di-tert-butyl-4-hydroxyphenylpropionyl)-trimethylendiamin, N,N'-Bis(3,5-di-tert-butyl-4-hydroxyphenylpropionyl)-hydrazin.
1.18. Ascorbinsäure (Vitamin C).
1.19. Aminische Antioxidantien, wie z.B. N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-lsopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-lsopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylaminophenol, 4-Nonanoylamino-phenol, 4-Dodecanoylamino-phenol, 4-Octadecanoylaminophenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/ lsohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethypiperidin-4-on, 2,2,6,6-Tetramethylpiperidin-4-ol.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,12-dihydroxy-3,7,10,14-tetrathiahexadecan.

Beispiele für Metalldesaktivatoren, z.B. für Kupfer, sind:
a) Benzotriazole und deren Derivate, z.B. 2-Mercaptobenzotriazol, 2,5-Dimercaptobenzotriazol, 4- oder 5-Alkylbenzotriazole (z.B. Tolutriazol) und deren Derivate, 4,5,6,7-Tetrahydrobenzotriazol, 5,5'-Methylenbis-benzotriazol; Mannich-Basen von Benzotriazol oder Tolutriazol wie 1-[Di(2-ethylhexyl)aminomethyl)-tolutriazol und 1-[Di(2-ethylhexyl)aminomethyl)-benzotriazol; Alkoxyalkylbenzotriazole wie 1-(Nonyloxymethyl)-benzotriazol, 1-(1-Butoxyethyl)-benzotriazol und 1-(1-Cyclohexybxybutyl)-tolutriazol.
b) 1,2,4-Triazole und deren Derivate, z.B. 3-Alkyl (oder Aryl)- 1,2,4-Triazole, Mannich-Basen von 1,2,4-Triazolen wie 1-[Di(2-ethylhexyl)aminomethyl-1,2,4-triazol; Alkoxyalkyl-1,2,4-triazole wie 1-(1-Butoxyethyl-1,2,4-triazol; acylierte 3-Amino-1,2,4-triazole.
c) Imidazolderivate, z.B. 4,4'-Methylenbis(2-undecyl-5-methylimidazol, Bis[(N-methyl)imidazol-2-yl]carbinol-octylether.
d) Schwefelhaltige heterocyclische Verbindungen, z.B. 2-Mercaptobenzothiazol, 2,5-Dimercapto-1,3,4-thiadiazol, 2,5-Dimercaptobenzothiadiazol und deren Derivate; 3,5-Bis[di(2-ethylhexyl)amino-methyl]-1,3,4-thiadiazolin-2-on.
e) Aminoverbindungen, z.B. Salicyliden-propylendiamin, Salicylaminoguanidin und deren Salze.

### Beispiele für Rost-Inhibitoren sind:

a) Organische Säuren, ihre Ester, Metallsalze, Aminsalze und Anhydride, z.B. Alkyl- und Alkenyl-bernsteinsäuren und deren Partialester mit Alkoholen, Diolen oder Hydroxycarbonsäuren, Partialamide von Alkyl- und Alkenylbernsteinsäuren, 4-Nonylphenoxyessigsäure, Alkoxy- und Alkoxyethoxycarbonsäuren wie Dodecyloxyessigsäure, Dodecyloxy(ethoxy)-essigsäure und deren Aminsalze, ferner N-Oleoyl-sarcosin, Sorbitan-mono-oleat, Blei-naphthenat, Alkenylbernsteinsäureanhydride, z.B. Dodecenylbernsteinsäure-anhydrid, 2-(2-Carboxyethyl)-1-dodecyl-3-methylglycerin und dessen Salze, insbesondere Na- und Triethanolaminsalze.
b) Stickstoffhaltige Verbindungen, z.B.:
   i. Primäre, sekundäre oder tertiäre aliphatische oder cycloaliphatische Amine und Amin--Salze von organischen und anorganischen Säuren, z.B. öllösliche Alkylammoniumcarboxylate, ferner 1-[N,N-bis-(2-hydroxyethyl)amino]-3-(4-nonylphenoxy)propan-2-ol.
   ii. Heterocyclische Verbindungen, z.B.: Substituierte Imidazoline und Oxazoline, 2-Heptadecenyl-1-(2-hydroxyethyl)-imidazolin.
c) Phosphorhaltige Verbindungen, z.B.: Aminsalze von Phosphorsäurepartialestern oder Phosphonsäurepartialestern, Zinkdialkyldithiophosphate.
d) Schwefelhaltige Verbindungen, z.B.: Barium-dinonylnaphthalin-sulfonate, Calciumpetroleum-sulfonate, Alkylthio-substituierte aliphatische Carbonsäuren, Ester von aliphatischen 2-Sulfocarbonsäuren und deren Salze.
e) Glycerinderivate, z.B.: Glycerin-monooleat, 1 -(Alkylphenoxy)-3-(2-hydroxyethyl)-glycerin, 1-(Alkylphenoxy)-3-(2,3-dihydroxypropyl)glycerin, 2-Carboxyalkyl-1,3-dialkylglycerin.

Beispiele für Viskositätsindex-Verbesserer sind: Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind: Poly(meth)acrylate, Ethylen-Vinylacetat-Copolymer, Alkylpolystyrole, Fumaratcopolymere, alkylierte Naphthalinderivate.

Beispiele für Dispergiermittel/Tenside sind: Polybutenylbernsteinsäureamide oder -imide, Polybutenylphosphonsäurederivate, basische Magnesium-, Calcium-, und Bariumsulfonate und -phenolate.

Beispiele für Hochdruck- und Verschleissschutz-Additive sind:
Schwefel- und/oder Phosphor- und/oder halogenhaltige Verbindungen wie z.B. chlorierte Paraffine, sulfurierte Olefine oder pflanzliche Öle (Soja-, Rapsöl), Alkyl- oder Aryl-di- oder -trisulfide, Zinkdialkyldithiophosphate, wie z.B. Zink-bis-(2-ethyl-hexyl)-dithiophosphat, Zinkdithiocarbamate wie z.B. Zinkdiamyldithiocarbamat, Molybdänphosphordithioate, Molybdän Dithiocarbamate, Triarylphosphate wie Tritolylphosphat, Tricresylphosphat, Phenylphosphatisopropylester, Aminsalze von Mono- oder Dialkylphosphorsäuren wie die Aminsalze von Mono/Di-hexylphosphat, Aminsalze von Alkylphosphonsäuren wie das Aminsalz der Methylphosphonsäure, Triarylphosphite wie z.B. Tris-[nonylphenyl]-phosphit, Dialkylphosphite, wie z.B. Dioctylphosphit, Triarylmonothiophosphate wie z.B. Triphenylthionophosphat oder Tris-[isononylphenyl]thionophosphat oder tert-butyliertes Triphenylthionophosphate, substitutierte Trialkylmono- oder dithiophosphate wie [(Diisopropoxyphosphinothioyl)thio]propionat oder Butylen-1,4-bis[(diisobutoxyphosphinothioyl)propionat], Trithiophosphate wie Trithiophosphorsäure S,S,S-tris(isooctyl-2-acetate), Aminsalze des 3-Hydroxy-1,3-thiaphosphetane-3-oxids, Benzotriazole oder deren Derivate wie bis(2-Ethylhexyl)aminomethyl tolutriazole, Dithiocarbamate wie Methylen-bis-dibutyldithiocarbamat, Derivate des 2-Mercaptobenzothiazols wie 1-[N,N-bis(2-ethylhexyl)aminomethyl]-2-mercapto-1H-1,3-benzothiazol, Derivate des 2,5-dimercapto-1,3,4-thiadiazols wie 2,5-bis(tert.nonylditdithio)-1 ,3,4-thiadiazol.

Beispiele für Reibwertverminderer, z.B. Öl aus Schmalz, Ölsäure, Talg, Rapsöl, geschwefelte Fette, Amine. Weitere Beispiele sind in *EP-A-565487genannt.*

Beispiele für besondere Additive zur Anwendung in Wasser/Öl-Metallbearbeitungs- und Hydraulikflüssigkeiten sind:
Emulgatoren: Petroleumsulfonate, Amine wie polyoxyethylierte Fettamine, nichtionische oberflächenaktive Substanzen; Puffer: Alkanolamine; Biocide: Triazine, Thiazolinone, Tris-Nitromethan, Morpholin, Natriumpyridenethol; Verarbeitungsgeschwindigkeitsverbesserer: Calcium- und Bariumsulfonate;
Beispiele für Treibstoffadditive:
Diese sind in *Kirk-Othmer, Encyclopedia of Chemical Technology, Vol. 12, 1994,* beschrieben. Hier handelt es sich im wesentlichen um Benzin- und Dieseladditive.
Benzin: Antioxidantien, aminisch, besonders para-Phenylendiamine oder phenolisch, z.B. 2,6-Di-tert.-butylphenol (wie weiter vorn beschrieben); Metalldesaktivatoren, besonders N,N'-Disalicyliden-1,2-propan, Benzotriazol, EDTA; Rostinhibitoren, beispielsweise Carbonsäuren, Sulfonate, Amine oder Aminsalze; Dispergatoren, z.B. Ester, Amine mit hohem Molekulargewicht, Mannich-Basen, Succinimide, borierte Succinimide; Detergentien, beispielsweise Fettsäureamide, nicht-polymere Amine, Polybutensuccinimide, Polyether-amine, niedermolekulare Amine, Sulfonate, Salicylsäurederivate; Demulgatoren, z.B. langkettige Alkohole oder Phenole mit Polyethylen oder -butylengruppen; Antiklopfmittel, z.B. Manganmethylcyclopentadienyltricarbonyl, Sauerstoffverbidungen, z.B. Ester von Pflanzenölen, Ether, Alkohole zur Verbesserung des Brennverhaltens.
Dieselkraftstoff: Zündverbesserer (Cetanverbesserer), z.B. Alkylnitrate, Ethernitrate, Alkyldiglycolnitrate, organische Peroxide; Stabilisatoren, insbesondere für Crackdiesel: Amine und andere N-haltige Verbindungen, die als Radikalfänger wirken; Rostschutzmittel wie weiter vorn beschrieben; Detergentien wie weiter vorn beschrieben; Sauerstoffverbindungen wie weiter vorn beschrieben; Kaltflussverbesserer: Das sind z.B. Stockpunkterniedriger wie weiter vorn beschrieben; Trübpunkterniedriger oder sog. Operability Additives (OA), das sind polymere Mehrkomponentensysteme, welche u.a. das Filterdurchflussverhalten verbessern.

Die Ausgangsprodukte sind an sich bekannt und/ oder ihre Herstellung ist in folgenden Beispielen beschrieben [z.B. Beispiele 1, 4, 6, 9, 11].

### Beispiele

Die folgenden Beispiele erläutern die Erfindung näher. Angaben in Teilen oder Prozent sind, sofern nicht anders angegeben, auf das Gewicht bezogen.

### Beispiel 1

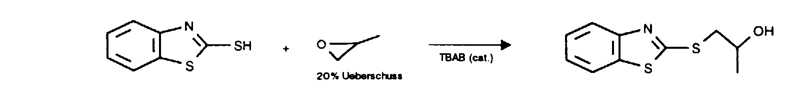

Zu einer Suspension von 172 g (1 Mol) 2-Mercaptobenzotriazolund 16,1 g (50 mmol) Tetrabutyl-ammoniumbromid in 900 ml Toluol werden bei 35°C innerhalb 30 min. 70 g (1,2 Mol) Propylenoxid zugetropft. Bei 40°C 4 Std. ausreagieren lassen. Danach mit zweimal ca. 100 ml 2N NaOH-Lösung extrahieren und mit fünfmal 100 ml Wasser waschen (→ pH 7-8). Die organische Phase eindampfen und bei reduziertem Druck (80°C/ ca. 0,03 mbar) trocknen. Man erhält 227 g klares, hellbraunes, mittelviskoses Öl (ca. 100 % d. Th.) [75235-71-1].

Elementaranalyse: 53,36 % C (berechnet 53,31); 4,92 % H (berechnet 4,92); 6,09 % N (berechnet 6,22); 28,56 % S (berechnet 28,46).

### Beispiel 2

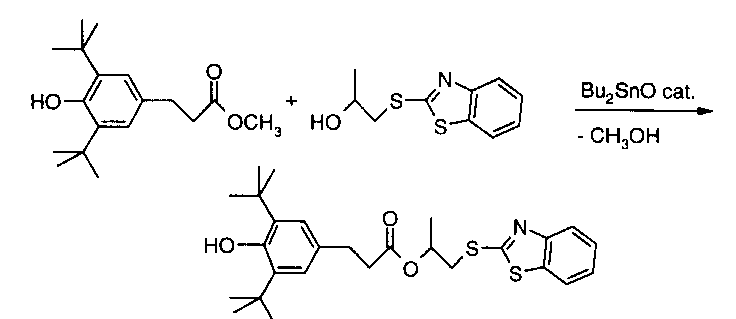

Zu einer Schmelze von 204,7 g (0,7 Mol) Metilox® (Ciba Specialty Chemicals) *), und 8,7 g (35 mmol) Dibutylzinnoxid werden bei 135-140°C innerhalb 2 Std. 166 g (0,7 Mol) Produkt aus Beispiel 1 [75235-71-1], gelöst in 100 ml Toluol, zugetropft und gleichzeitig Toluol und Methanol abdestilliert. Nach beendeter Zugabe 4 Std. bei 135-140°C reagieren lassen, bei gleichzeitigem Zutropfen und Abdestillieren von insgesamt ca. 50 ml Toluol. Nach Zugabe von weiteren 3,5 g (14 mmol) Dibutylzinnoxid weitere 4 Std. bei 135-140°C (+ Toluol,-Toluol/Methanol) ausreagieren lassen. - Nicht umgesetztes METILOX (ca. 103 g, 0,35 Mol) wird bei reduziertem Druck abdestilliert (140°C / ca. 0,03 mbar). Das verbleibende gelbe, mittelviskose Oel wird in ca. 400 ml Siedegrenzbenzin (Kp. 80 -110°C) gelöst, mit zweimal ca. 100 ml 2N NaOH-Lösung extrahiert und mit dreimal ca. 100 ml Wasser gewaschen (→ pH 7). Die organische Phase wird durch wenig Filterhilfsmittel (Hyflo®) klarfiltriert, eingedampft und bei reduziertem Druck (140°C/ ca. 0,03 mbar) getrocknet. Man erhält 224 g viskoses, hellorange-farbenes, klares Öl (66 % d. Th.).
Elementaranalyse: 64,75 % C (berechnet 66,77); 7,42 % H (berechnet 7,26);
3,22 % N (berechnet 2,88); 14,26 % S (berechnet 13,20).
*) β-(3,5-Di-tert-butyl-4-hydroxyphenyl)-propionsäure-methyl-ester [6386-38-5].

### Beispiel 3

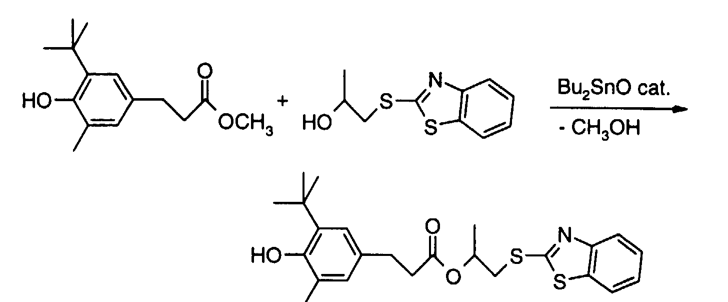

In gleicher Weise wie in Beispiel 2 werden 22,15 g (90 mmol) Methyl-METILOX *) mit 20,3 g (90 mmol) Produkt aus Beispiel 1 [75235-71-1] in Gegenwart einer katalytischen Menge an Dibutylzinnoxid (1,12 g + 0,6 g, 6,9 mmol) umgesetzt: Man erhält 27,9 g braunes, dickflüssiges Öl (70 % d. Th.).
Elementaranalyse: 64,51 % C (berechnet 64,83); 6,76 % H (berechnet 6,80);
3,2 % N (berechnet 3,15); 14,06 % S (berechnet 14,42).
*) β-(5-tert-Butyl-4-hydroxy-3-methylphenyl)-propionsäuremethylester [6386-39-6].

### Beispiel 4

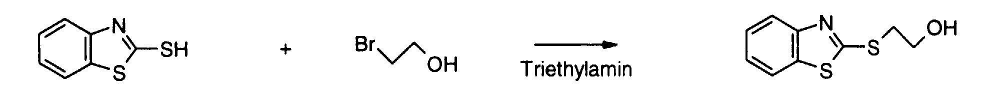

Zu einer Emulsion von 172 g (1,0 Mol) 2-Mercaptobenzothiazol und 121,4 g (1,2 Mol) Triethylamin in 1 l Toluol werden bei 30-40°C innerhalb 1,5 Std. 125 g (1,0 Mol) 2-Bromethanol zugetropft. Dann wird 1,5 Std. bei 30°C und 2.5 Std. bei 70 C weitergerührt. Die feine Suspension wird mit zweimal 500 ml Wasser, 200 ml 2N NaOH-Lösung und nochmals Wasser gewaschen (pH 7), eingedampft und bei reduziertem Druck (100°C/ca. 0.03 mbar) getrocknet: Man erhält 192 g braunes Öl (91% d. Th.). Dieses Rohprodukt [4665-63-8] wird direkt weiterumgesetzt (z.B. in Beispiel 5).

### Beispiel 5

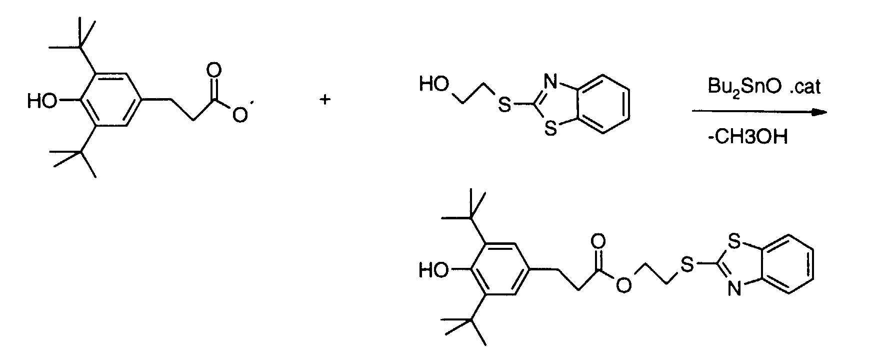

In gleicher Weise wie in den Beispielen 2 und 3 werden 26,3 g (90 mmol) METILOX mit 19 g (90 mmol) Produkt aus Beispiel 1 in Gegenwart einer katalytischen Menge Dibutylzinnoxid (1,7 g, 7 mmol) umgesetzt. Das Rohprodukt wird chromatographisch über 200 g Silicagel (Toluol/Ethylacetat) vorgereinigt und aus Ethanol (ca.190 ml) umkristallisiert: Man erhält 22 g weisses Kristallisat; Smp. 108-111°C (52% d. Th.).
Elementaranalyse: 66,00 % C (berechnet 66,21); 7,29 % H (berechnet 7,05);
2,94 % N (ber. 2,97); 13,59 % S (berechnet 13,59).

### Beispiel 6

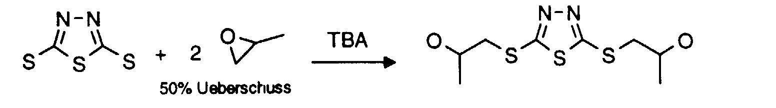

Zu einer Suspension von 66 g (0,44 Mol) 2,5-Dimercapto-1,3,4-thiadiazol [1072-71-5] und 7,1 g (22 mmol) Tetrabutyl-ammoniumbromid (TBAB) in 270 ml Tetrahydrofuran werden bei 30-35°C innerhalb 1 Std. eine Lösung von 76,7 g (1,32 Mol) Propylenoxid in 50 ml Tetrahydrofuran zugetropft. Anschliessend bei 40°C 1 Std. ausreagieren lassen. Die dunkle Lösung wird eingedampft und das dunkle Öl (ca. 130 g) wird chromatographisch durch 500 g Sillicagel eluiert (Toluol/ Ethylacetat). Die Fraktionen des Hauptproduktes werden eingedampft und bei reduziertem Druck (120°C/ ca. 0,03 mbar) getrocknet: Man erhält 97,0 g orangefarbenes, viskoses Oel (83 % d. Th.) [107641-99-6].
Elementaranalyse: 36,50 % C (berechnet 36,07), 5,34 % H (berechnet 5,30);
10,58 % N (berechnet 10,52), 36,52 % S (berechnet 36,10).

### Beispiel 7

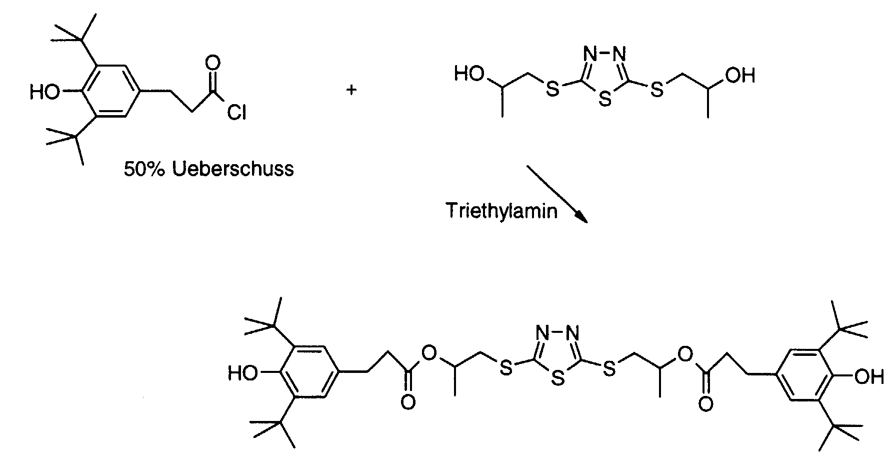

Zu einer Lösung von 16 g (60 mmol) Produkt aus Beispiel 6 [107641-99-6] und ca. 53 g (180 mmol) METILOX-Säurechlorid *) in 240 ml Toluol lässt man bei 5-10°C innerhalb 25 Min. ca. 25 ml (180 mmol) Triethylamin zutropfen und lässt bei 30-40°C 4,5 Std. ausreagieren. Die trübe orangefarbene Reaktionsmischung mit 500 ml Wasser, 100 ml 2N-NaOH-Lösung und nochmals Wasser (→ pH 7-8) waschen und eindampfen. Das Rohprodukt (ca. 70 g) wird säulenchromatographisch über 500 g Silicagel (Hexan/ Toluol/Ethylacetat) gereinigt. Die Fraktionen des Hauptproduktes werden eingedampft und bei reduziertem Druck (120°C/ ca. 0,03 mbar) getrocknet: Man erhält 17,8 g gelbes, klebriges Harz (38 % d. Th.).
Elementaranalyse: 64,42 % C (berechnet 64,09); 8,06 % H (berechnet 7,94);
3.47 % N (berechnet 3,56); 11,74 % S (berechnet 12,22).
*) β-(3,5-Di-tert-butyl-4-hydroxyphenyl)-propionsäure-chlorid [3062-64-4].

### Beispiel 8

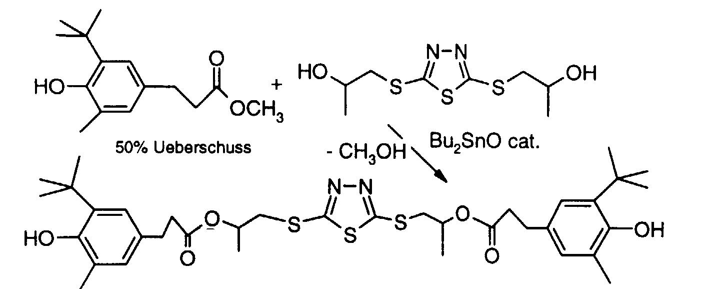

In analoger Weise wie in den Beispielen 2, 3 und 5 erhält man durch katalysierte Umesterung von 2 Äquivalenten Methyl-METILOX *) mit dem Produkt aus Beispiel 6 das tert.-Butyl/Methyl-Analoge zu Beispiel 7: Man erhält 8,0 g braunes, klebriges Harz (ca. 20% d. Th.).
Elementaranalyse: 61,04 % C (berechnet 61,51); 7,45 % H (berechnet 7,17);
3,94 % N (berechnet 3,99); 13,48 % S (berechnet 13,68).
*) β-(5-tert-Butyl-4-hydroxy-3-methylphenyl)-propionsäuremethylester [6386-39-6].

### Beispiel 9

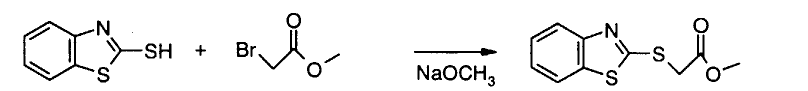

Zu einer Lösung von 88 g (0.5 Mol) 2-Mercaptobenzothiazol und 108 g Natriummethanolat 30 % (0.6 Mol) in 850 ml Methanol werden bei 0-5°C innerhalb 1 Std. tropfenweise 94,6 g (0,6 Mol) Bromessigsäuremethylester zugeben. Bei Raumtemperatur wird 1 Std. nachgerührt. Dann wird eingedampft, wieder in 400 ml Toluol gelöst und mit dreimal 100 ml gesättigter Natriumchloridlösung neutral gewaschen (pH ca. 6). Die Toluol-Phase wird eingedampft: Man erhält 131 g hell-orangefarbenes Oel. Dieses Rohprodukt wird aus Hexan (ca. 1200 ml) umkristallisiert : 102 g weisses Kristallisat; Smp. 74-76°C (86% d. Th.) [24044-87-9].
Das Produkt wird in dieser Qualität weiter umgesetzt (z.B. in Beispiel 14).

### Beispiel 10

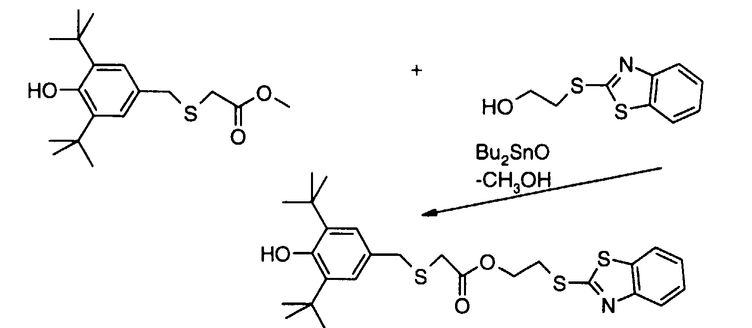

In gleicher Weise wie in den Beispielen 2, 3, 5 und 8 werden 29,4 g (90 mmol) 3,5-Bis(tert-butyl)4-hydroxybenzylthioessigsäuremethylester [51511-20-7] mit 16,9 g (80 mmol) Produkt aus Beispiel 1[4665-63-8] in Gegenwart einer katalytischen Menge Dibutylzinnoxid (0,5 g, 9 mmol) umgesetzt. Das Rohprodukt wird zweimal chromatographisch über 550 g resp. 300 g Silicagel (Hexan - Toluol - Ethylacetat) gereinigt. Man erhält 8,8 g gelbes, sehr viskoses Oel (22 % d. Th.).
Elementaranalyse : 62,48 % C (berechnet 61,99); 7,01 % H (berechnet 6,60);
2,75 % N (berechnet 2,78); 19,02 % S (berechnet 19,09).

### Beispiel 11

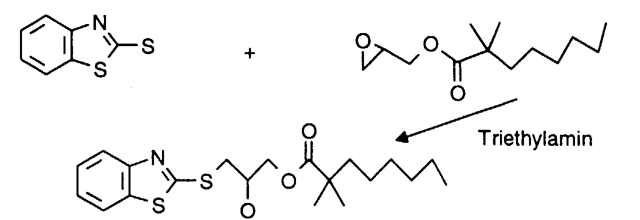

Zu einer Suspension von 172 g (1,0 Mol) 2-Mercaptobenzothiazol und 5ml Triethylamin in 1,5 l Toluol werden bei 60°C innert 30 min. 253 g (1,0 Mol) Glydexx® (Exxon) N 10 [Exxon Chemicals: 52636-92-7] zugetropft. Dann wird innerhalb 15 min. auf 80°C erwärmt und 3 Std. bei dieser Temperatur weitergerührt. Die Toluol-Lösung wird dann mit zweimal 50 ml 1N NaOH-Lösung und viermal mit 50ml Wasser neutral gewaschen (pH 7). Die organische Phase wird durch wenig Filterhilfsmittel (HYFLO) klarfiltriert, eingedampft und bei reduziertem Druck (100°C/ ca. 0,03 mbar) getrocknet: Man erhält 405 g (ca. 100 % d. Th.) klares, mittelviskoses Oel; n_{D}²⁰: 1,5578.
Elementaranalyse: 60,95% C (berechnet 60,73); 7,63 % H (berechnet 7,39);
3,53 % N (berechnet 3,54); 15,80 % S (berechnet 16,21).

### Beispiel 12

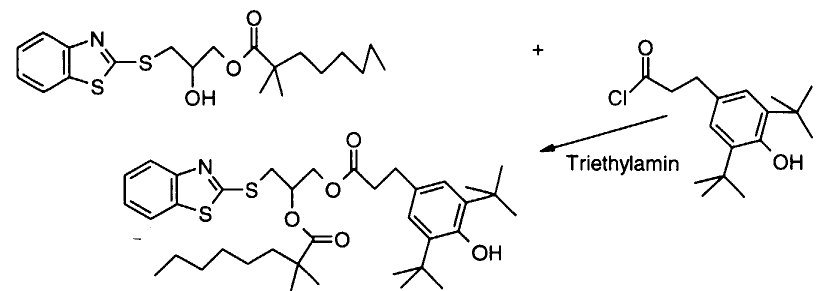

Zu einer Lösung von 23,7 g (60 mmol) Produkt aus Beispiel 11 und 18,7 g (63 mmol) METILOX-Säurechlorid [3062-64-4] in 200ml Toluol werden bei 0-10°C innerhalb 15 min. 6,7 g (66 mmol) Triethylamin zugetropft. Bei Raumtemperatur wird 3 Std. weitergerührt. Dann wird die braune Suspension filtriert. Das Filtrat wird mit einmal 100ml 2N-Natrium-hydroxid-Lösung und Wasser neutral gewaschen, und eingedampft. Das Rohprodukt (ca. 45g) wird chromatographisch über 500 g Silicagel (Hexan/ ToluoV Ethylacetat) gereinigt. Die Fraktionen mit dem Hauptprodukt werden eingedampft und bei reduziertem Druck getrocknet. (100°C/ ca.0,03 mbar) : 21 g orangefarbenes, sehr viskoses Oel (53% d. Th.).
Elementaranalyse : 67,99 % C (berechnet 67,75); 8,30 % H (berechnet 8,14);
2,07 % N (berechnet 2,14); 8,75 % S (berechnet 9,78).

### Beispiel 13

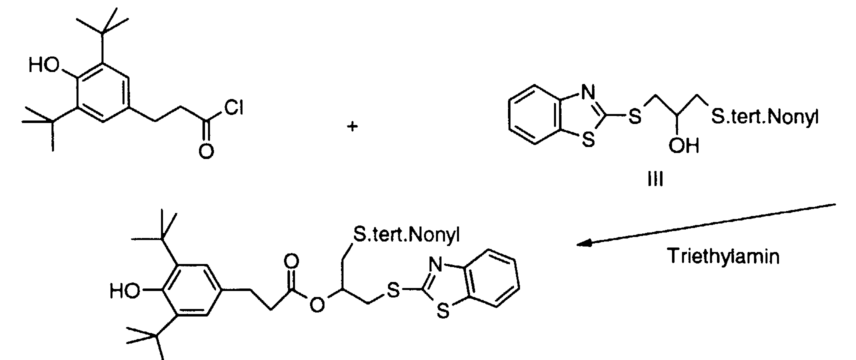

Zu einer Lösung von 38,4g (0,1mol) III *) und 31,2g (0,105mol) METILOX-Säurechlorid [3062-64-4] in 200 ml Toluol bei 0-10°C innerhalb 25 min. 11,1g (0,11mol) Triethylamin zutropfen. Anschliessend bei 30°C noch 2 Std. ausreagieren lassen. Bei Raumtemperatur mit 100 ml Hexan verdünnen, filtrieren und eindampfen. Das Rohprodukt (braunes Oel, ca. 72 g) wird säulenchromatographisch über ca. 500 ml Silicagel (Hexan/ Toluol/ Ethylacetat) gereinigt. Die Fraktionen des Hauptproduktes werden eingedampft und bei reduziertem Druck (100°C/ ca. 0,03 mbar) getrocknet. Man erhält 46,2g sehr viskoses gelbes Oel (72 % d. Th.).
Elementaranalyse: 66,7 % C (berechnet 67,14); 8,49 % H (berechnet 8,30);
2,28% N (berechnet 2,17); 15,03 % S (berechnet 14,93).
*) siehe EP 166 696, Bsp.13 (mit tert.-Dodecyl)

### Beispiel 14

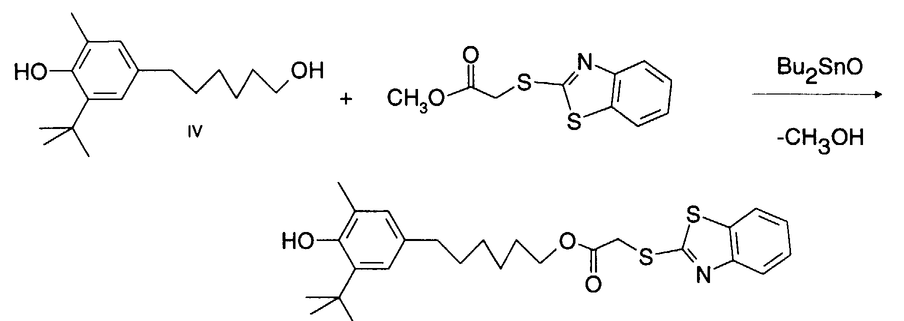

Eine Suspension von 23,8g (90 mmol) IV *), 21,5 g (90 mmol) des Produktes aus Beispiel 9 **) und 1,7 g Dibutylzinnoxid in 50 ml Toluol wird langsam auf 135 - 140°C erwärmt, bei gleichzeitigem Abdestillieren des Toluols. Dann wird bei dieser Temperatur weiterhin Toluol tropfenweise zugegeben und mit dem sich abspaltenden Methanol abdestilliert. Nach ca. 4 Std. werden weitere 1,7 g Dibutylzinnoxid zugegeben und nochmals nach 5 Std.. Nach 8 Std. wird eingedampft und säulenchromatographisch über 500 g Silicagel (ToluoV Ethylacetat) gereinigt. Die Fraktionen des Hauptproduktes werden eingedampft und bei reduziertem Druck (100°C/ ca. 0,03 mbar, ca. 30 min.) getrocknet: Man erhält 9,4 g sehr viskoses gelbes Oel (22% d. Th.).
Elementaranalyse: 66,53 % C (berechnet 66,21); 7,29 % H (berechnet 7,05);
2,95 % N (berechnet 2,97); 13,71 % S (berechnet 13,59).
*) [152211-02-4]; **)[24044-87-9].

### Beispiel 15

Test auf Verschleissschutz: Zur Prüfung auf die Eignung als Verschleissschutzadditiv wird die ASTM-Standardmethode D-2783-81 unter Verwendung des Shell-Vierkugel-Apparates herangezogen. Als Basisöl wird Stock 305 der Fa. Mobil verwendet, dem die in Tabelle I angegebene Menge an Verbindung gemäss dem jeweils genannten Beispiel zugegeben wird. Ermittelt werden der mittlere Verschleiss-Narben-Durchmesser WSD (Wear-Scar-Diameter, in mm) bei einer Last von 40 kg und einer Rotationsgeschwindigkeit von 1440 Upm nach 1 Std. Betrieb bei 100°C. Die gemessenen Resultate sind in Tabelle I aufgeführt.

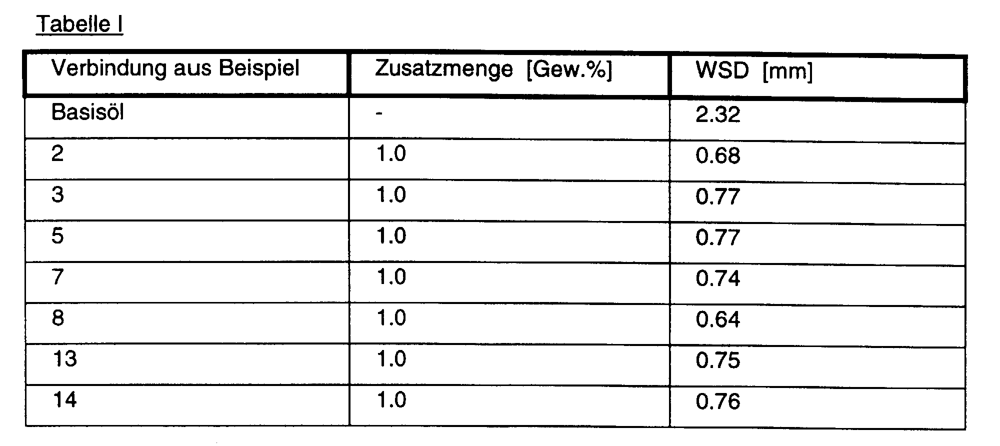

## Patentansprüche

1. Verbindungen der Formeln: 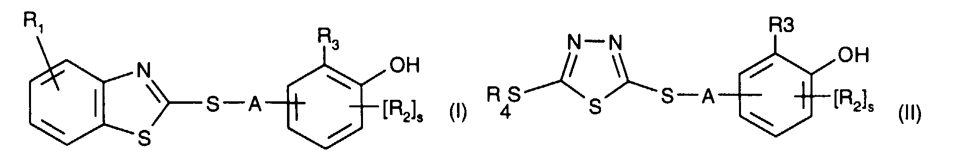
mit den Bedeutungen für:
R₁ = Wasserstoff oder C₁-C₂₀-Alkyl;
R₂ = Wasserstoff, C₁-C₁₈-Alkyl, C₅-C₁₂-Cycloalkyl, C₇-C₁₀-Bicycloalkyl, Phenyl oder C₇-C₉-Phenylalkyl;
R₃ = C₁-C₁₈-Alkyl, C₅-C₁₂-Cycloalkyl, Phenyl oder C₇-C₉-Phenylalkyl;
s = 0,1 oder 2;
R₄ = C₅-C₂₀-Alkyl, C₅-C₁₂-Cycloalkyl, Phenyl, Naphthyl, C₇-C₉-Phenylalkyl, gegebenenfalls unterbrochen durch ein oder mehrere bivalente Reste aus der Gruppe -O-, -NR₅-, -S-, -C(=O)-O-, -O-C(=O)-, -C(=O)-NR₅- und -NR₅-C(=O)-, oder substituiert durch ein oder mehrere Reste der Gruppe -OH, -NH₂ und -C(=O)-OH oder bedeutet -A-[(C₆H₂)R₂(OH)R₃];
R₅ = H oder C₁-C₁₈-Alkyl;
A = C₁-C₂₅-Alkylen, gegebenenfalls unterbrochen durch ein oder mehrere bivalente Reste aus der Gruppe -O-, -NR₅-, -S-, -C(=O)-O-, -O-C(=O)-, -C(=O)-NR₅- und -NR₅-C(=O)-, oder substituiert durch ein oder mehrere Reste der Gruppe -OH, -NH₂ und -C(=O)-OH, wobei mindestens eine Einheit -C(=O)-O- oder -O-C(=O)- enthalten ist.

2. Verbindungen gemäss Anspruch 1 mit den Bedeutungen für
R₁ = Wasserstoff;
R₂ = Wasserstoff, C₁-C₈-Alkyl, C₅-C₆-Cycloalkyl, Phenyl, Benzyl oder 2-Norbornyl;
R₃ = C₁-C₈-Alkyl, C₅-C₆-Cycloalkyl, Phenyl, Benzyl oder 2-Norbornyl;
s = 0,1 oder 2;
R₄ = C₅-C₇-Cycloalkyl, Phenyl, Benzyl, C₁-C₁₈-Alkyl, gegebenenfalls unterbrochen durch ein oder mehrere bivalente Reste aus der Gruppe -O-, -NH-, -S-, -C(=O)-O-, -O-C(=O)-, -C(=O)-NR₅- und -NR₅-C(=O)-,
oder substituiert durch ein oder mehrere Reste der Gruppe -OH und -C(=O)-OH oder bedeutet -A-[(C₆H₂)R₂(OH)R₃];
R₅ = H oder C₁-C₈-Alkyl;
A = C₁-C₁₈-Alkylen, gegebenenfalls unterbrochen durch ein oder mehrere bivalente Reste aus der Gruppe -O-, -NR₅-, -S-, -C(=O)-O-, -O-C(=O)-, -C(=O)-NR₅- und -NR₅-C(=O)-, oder substituiert durch ein oder mehrere Reste der Gruppe -OH, -NH₂ und -C(=O)-OH, wobei mindestens eine Einheit -C(=O)-O- oder -O-C(=O)- enthalten ist.

3. Verbindungen gemäss Anspruch 1 mit den Bedeutungen für
R₁ = H; R₂ = Methyl oder tert-Butyl; R₃ = tert-Butyl;
s = 0, 1 oder 2;
R₄ = -A-[(C₆H₂)(Methyl oder tert.-Butyl)(OH)(tert.-Butyl)];
A = -CH₂CH₂O-(C=O)-CH₂CH₂- oder -CH₂CH(CH₃)-O-(C=O)-CH₂CH₂-.

4. Verbindungen der Formeln 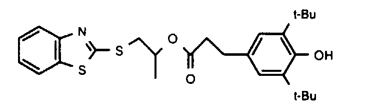 und

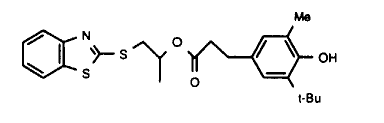

5. Verbindungen der Formeln: 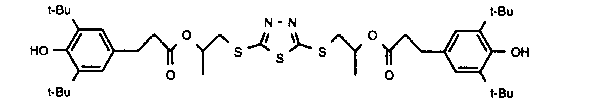 und

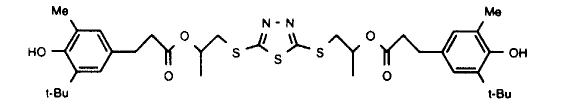

6. Zusammensetzungen enthaltend eine Verbindung der Formel I oder II oder Mischungen davon in Kombination mit einem Grundöl mit schmierender Viskosität oder Kraftstoffen.

7. Konzentrate enthaltend ein Öl und mindestens eine Verbindung gemäss Anspruch 1.

8. Verfahren zur Verbesserung der Gebrauchseigenschaften von Schmierstoffen oder Schmierfetten oder flüssigen Kraftstoffen, gekennzeichnet durch die Zugabe mindestens einer Verbindung der Formel I oder II gemäss Anspruch 1.

9. Verwendung von Verbindungen gemäss Anspruch 1 als Additive in Schmierstoffen, Hydraulik- oder Metallbearbeitungsflüssigkeiten sowie Kraftstoffen.
